# EUROPÄISCHE PATENTSCHRIFT

(11) **EP 2 298 399 B1**
(45) Veröffentlichungstag und Bekanntmachung des Hinweises auf die Patenterteilung: **10.02.2016**
(21) Anmeldenummer: 09170436.1
(22) Anmeldetag: 16.09.2009
(51) Int. Cl.: A61M 16/01, A61M 16/00, A61M 16/22

(54) **Anästhesievorrichtung und Verfahren zum Betreiben einer Anästhesievorrichtung**
Anaesthetic device and method for operating same
Dispositif d'anesthésie et procédé de fonctionnement d'un dispositif d'anesthésie

(43) Veröffentlichungstag der Anmeldung: 23.03.2011
(73) Patentinhaber: Drägerwerk AG & Co. KGaA, 23558 Lübeck (DE)
(72) Erfinder: Heesch, Ralf, 23568, Lübeck (DE)
(74) Vertreter: UEXKÜLL & STOLBERG

(56) Entgegenhaltungen:
- WO-A1-2008/000299
- DE-U1- 9 407 517
- US-A1- 2006 174 889

## Beschreibung

Die vorliegende Erfindung bezieht sich auf eine Anästhesievorrichtung, mit der ein in einem Atemkreis befindliches Atemgas, welches insbesondere mit einem Narkose- bzw. Anästhesiemittel angereichert ist, schnell und kostengünstig ausspült werden kann. Die Erfindung ist bevorzugt für die Ausspülung und die Trocknung des Atemkreises der Anästhesievorrichtung geeignet, um mit einer passenden Steuereinrichtung den Atemkreis automatisch oder manuell von einem Anästhesisten oder anderen medizinischem Personal zwischen zwei Patientenbehandlungen aufzubereiten. Insbesondere bezieht sich die Erfindung auf ein sogenanntes Rückatemsystem, in dem CO₂ von dem ausgeatmeten Atemgas mittels eines CO₂-Absorbers entfernt wird und das verbleibende Atemgas wieder dem inspiratorischen Atemgas hinzugefügt werden kann. Ein Atemkreis wird typischerweise in einer von zwei Betriebsarten betrieben: einer halb geschlossenen Betriebsart oder als weitestgehend geschlossener Kreis.

Bei der halb geschlossenen Betriebsart übersteigt die Menge des Atemgases diejenige, die von einem Patienten aufgenommen wird. Das überschüssige Atemgas wird aus dem Atemkreis in ein Narkosefortleitungssystem während der Ausatmungsphase des Patienten abgegeben. In der Betriebsart des geschlossenen Atemkreises werden die Atemgase durch den CO₂-Absorber im Wesentlichen wieder aufbereitet. Dazu wird ein geeignetes Absorptionsmittel wie beispielsweise Natronkalk verwendet. Der Atemkreis umfasst typischerweise einen inspiratorischen Atemgaskanal und einen exspiratorischen Atemgaskanal zur Bereitstellung von Atemgas für den Patienten. Das Atemgas wird dabei mittels eines Anästhesiemittel-Dosierers mit Anästhesiemittel angereichert. Der inspiratorische Atemgaskanal und der exspiratorische Atemgaskanal sind über ein sogenanntes Y-Stück miteinander verbunden, welches über einen angeschlossenen Schlauch der Weiterleitung von Atemgas an den Patienten dient. In einem Atemkreis befinden sich bis zu fünf Liter Atemgas. Zwischen zwei Patientenbehandlungen kann sich in dem Atemkreis noch Atemgas des vorhergehenden Patienten befinden. In der Regel lassen sich die Atemkreise der Anästhesievorrichtungen nur schlecht pneumatisch ausspülen. Insbesondere kann es bei nachfolgenden Patienten zu einer Unverträglichkeit gegenüber dem bereits verwendeten Atemgas kommen. Eine aufwendige Aufbreitung des Atemkreises mit Hilfe einer angeschlossenen Simulationslunge bei gleichzeitiger Zuführung von Atemgas aus der Atemgasversorgung ist in der Regel dafür erforderlich. Zur Sicherstellung, dass sich kein Anästhesiemittel in den Elementen des Atemkreises befindet, muss somit der Atemkreis der Anästhesievorrichtung komplett gespült werden. Insbesondere ist diese Maßnahme bedeutsam für den Fall, dass ein anderes Anästhesiemittel für den nächsten Patienten genutzt werden soll.

Aus U.S. 2006/174889 A1 ist eine Anästhesievorrichtung mit den Merkmalen des Oberbegriffs von Anspruch 1 bekannt. Darin ist eine Atemgasversorgung für die Bereitstellung von Atemgas an einem Patienten während einer Operation beschrieben, insbesondere für die Bereitstellung von Anästhesiegas. In der beschriebenen Vorrichtung, die in Fig. 7 dargestellt ist, wird mit einer zusätzlichen Gasversorgungsquelle ein zusätzliches Atemgas zur Verfügung gestellt. In dem dargestellten Beispiel der Fig. 7 besteht das zusätzliche Gas aus Luft. Das Gas wird durch ein unidirektionales Ventil in den Atemkreis befördert, wobei ein Teil des Gases zum Patienten geführt wird. Ein Teil der bereitgestellten Luft wird dabei kontinuierlich dem Patienten zur Verfügung gestellt. Ein Gebläse zur Einleitung der Luft in den Atemkreis befindet sich in einem Nebenstrom des Atemkreises.

In der WO 2008/000 299 A1 ist sowohl eine Anästhesievorrichtung als auch ein Verfahren zum Betreiben der Anästhesievorrichtung offenbart, mit dem mittels einer Kontrolleinrichtung ein Gasfluss eines Spülgases aus einer Atemgasversorgung gesteuert wird. Es kann hierbei eine Spülung des Anästhesiemittels aus der Anästhesievorrichtung erfolgen, wenn die Anästhesievorrichtung nicht mit einem Patienten verbunden ist. Hierfür wird typischerweise Gas aus der zentralen Gasversorgung eines Krankenhauses verwendet. Die Verwendung dieses technische Atemgas für die Wiederaufbereitung des Atemkreises ist sehr kostenintensiv.

Es ist daher Aufgabe der vorliegenden Erfindung, eine Anästhesievorrichtung zur Beatmung von Patienten zur Verfügung zu stellen, mit deren Hilfe die vorstehend genannten Nachteile überwunden werden. Es ist insbesondere Aufgabe der Erfindung, ein Verfahren zum Betreiben der Anästhesievorrichtung zur Verfügung zu stellen, mit Hilfe dessen eine einfache Aufbereitung der Anästhesievorrichtung zwischen den einzelnen Patienten erfolgen kann.

Diese und weitere Aufgaben werden durch eine Anästhesievorrichtung mit den Merkmalen des Patentanspruchs 1 und ein Verfahren mit den Merkmalen des Patentanspruchs 8 gelöst.

In den abhängigen Patentansprüchen sind vorteilhafte und bevorzugte Weiterbildungen der erfindungsgemäßen Anästhesievorrichtung sowie des erfindungsgemäßen Verfahrens angegeben.

Ein wesentlicher Vorteil der erfindungsgemäßen Anästhesievorrichtung besteht darin, dass für eine Aufbereitung zwischen den einzelnen Patientenbehandlungen keine kostenintensiven technischen Atemgase, welche von der zentralen Gasversorgung bereitgestellt werden, erforderlich sind. Mittels der Umgebungsluft, welche durch einen Lufteinlass in dem Atemkreis von der Atemkreisfördereinrichtung angesaugt wird, kann das Atemgas aus dem Atemkreis in vorteilhafter Weise befördert werden. Der Atemkreis der erfindungsgemäßen Anästhesievorrichtung umfasst dabei einen inspiratorischen Atemgaskanal und einen exspiratorischen Atemgaskanal, ein verschließbar ausgeführtes Y-Stück zur Verbindung des inspiratorischen und des exspiratorischen Atemgaskanals mit dem Patientenzugang. Das Y-Stück wird typischerweise mittels eines Dichtungsstopfens verschlossen. Ferner ist eine zentrale Gasversorgung zur Einleitung von Atemgas in den Atemkreis, eine Atemgasfördereinrichtung, ein Atemgasauslasskanal mit einem Atemgasauslassventil, ein Atemgasreservoir zur Zwischenspeicherung von Atemgas, ein dem Atemgasreservoir vorgeschaltetes Peep-Ventil, ein Lufteinlass zur Einleitung von Umgebungsluft in den Atemkreis und eine Steuereinrichtung vorgesehen Die Steuereinrichtung dient wenigstens zur Steuerung der durch den Lufteinlass eintretenden Umgebungsluft zur Reinigung des Atemkreises von Atemgasen bei einem patientenseitig geschlossenen Y-Stück. In einer bevorzugten Ausgestaltung ist der Lufteinlass dem Atemgasreservoir nachgeschaltet im Atemkreis angeordnet. Somit kann in vorteilhafter Weise gemäß den erfindungsgemäßen Verfahrensschritten nach Schließen des Y-Stückes und des Peep-Ventils, Öffnen des Atemgasauslassventils und des Lufteinlasses und nach einem Start der Atemgasfördereinrichtung ein Unterdruck erzeugt werden, der eine Strömung des in dem Atemgasreservoir befindlichen Atemgases durch den Atemgaskreis zu einem Atemgasauslasskanal bewirkt.

In vorteilhafter Weise kann damit mittels Umgebungsluft wenigstens ein erster Teil des inneren Atemkreises der Anästhesievorrichtung von Atemgas gereinigt werden. In einer weiteren vorteilhaften Ausbildung der erfindungsgemäßen Anästhesievorrichtung ist ein erster Volumenstrom-Sensor im Atemkreis, vorzugsweise im exspiratorischen Atemgaskanal, vorgesehen. Der erste Volumenstromsensor misst den Volumenstrom nach dem Start der Atemgasfördereinrichtung. Nach dem Erreichen eines vorherbestimmten Atemgasvolumens wird das Atemauslassventil geschlossen und das Peep-Ventil geöffnet. Damit wird auch der restliche Teil des inneren Atemkreises der Anästhesievorrichtung in einer zweiten Phase von Atemgas befreit.

Eine Umschaltung von der ersten Phase des erfindungsgemäßen Verfahrens in eine weitere vorteilhafte Ausbildung des erfindungsgemäßen Verfahrens einer zweiten Phase erfolgt bei einem Volumenstromwert, der im Wesentlichen dem Volumen des in der ersten Phase zu reinigenden Teils des Atemkreises entspricht. Das Volumen liegt hierbei im Bereich von vorzugsweise 10 bis 15 Liter. Die Atemgasfördereinrichtung arbeitet in vorteilhafter Weise bei einer Drehzahl von vorzugsweisen 50 bis 100 Liter pro Minute.

Alternativ zur Messung des Volumenstroms kann als Kriterium für eine Umschaltung von der ersten in die zweite Phase eine Zeit gemessen werden. Die Zeit beträgt dabei vorzugsweise 15 - 45 Sekunden.

In einer weiteren Ausbildung der erfindungsgemäßen Anästhesievorrichtung ist ein zweiter Volumenstromsensor im Atemkreis, vorzugweise im inspiratorischen Atemgaskanal, vorgesehen. Ein ordnungsgemäßes Schließen des Y-Stückes gemäß dem Verfahrensschritt A kann durch eine Bildung der Differenz der Volumenstromwerte des ersten und zweiten Volumenstromsensors überprüft und alarmiert werden.

Eine noch im Atemkreis vorhandene Konzentration von Anästhesiemittel kann ferner mittels eines vorzugsweise zwischen Y-Stück und Lufteinlass angeordnetem Gasmessmodul überprüft werden. Entsprechend der noch vorhandenen Konzentration von Anästhesiemittel sind die Verfahrensschritte A bis E des erfindungsgemäßen Verfahrens beliebig oft wiederholbar. Alternativ hierzu kann vom Anwender eine Zeitdauer zur Durchführung des erfindungsgemäßen Verfahrens gewählt werden.

Die erfindungsgemäße Anästhesievorrichtung kann weiter einen CO₂-Absorber in dem Atemkreis vorsehen, welcher vorzugsweise dem Atemgasreservoir nachgeschaltet angeordnet ist. Mittels eines CO₂-Absorbers erfolgt eine Trennung des vom Patienten ausgeatmeten Atemgases von CO₂. Bei dieser Trennung wird unter anderem Feuchtigkeit und Wärme produziert. Gemeinsam mit der von dem Patienten ausgeatmeten Feuchtigkeit kann es häufig zu einem Auskondensieren innerhalb des Atemkreises kommen. Die Funktionalität des Atemkreises kann aufgrund einer Akkumulierung dieses Kondensats über mehrere aufeinanderfolgende Anwendungen beeinträchtigt werden. Zum Entfernen dieser Feuchtigkeit kann in einem weiteren Schritt des erfindungsgemäßen Verfahrens durch mehrfache Wiederholung der Verfahrensschritte ein Trocknungseffekt des Atemkreises erreicht werden. Diese sogenannte Trocknungsphase sichert ab, dass der Kalk innerhalb des CO₂-Absorbers aufgrund der angewendeten Umgebungsluft nicht komplett austrocknet. Zusätzlich kann eine Heizung innerhalb des Atemkreises vorgesehen sein, die in vorteilhafter Weise mit einer höheren Heizleistung betrieben wird als im normalen Patientenbeatmungsmodus. Dies führt in vorteilhafter Weise zu einer Beschleunigung der Austrocknung.

Die vorliegende Erfindung wird nun anhand eines Beispiels unter Bezugnahme auf die Zeichnungen beschrieben.

Dabei zeigen:
- Figur 1: eine schematische Darstellung eines Atemkreises des erfindungsgemäßen Anästhesiesystems,
- Figur 2: eine schematische Darstellung des erfindungsgemäßen Anästhesiesystems in der Durchführung des erfindungsgemäßen Verfahrens in einer ersten Ausführung,
- Figur 3: eine schematische Darstellung des erfindungsgemäßen Anästhesiesystems in der Durchführung des erfindungsgemäßen Verfahrens in einer zweiten Ausführung und
- Figur 4: eine schematische Darstellung des erfindungsgemäßen Anästhesiesystems in einer weiteren Ausführung des erfindungsgemäßen Verfahrens (Trocknungsfunktion).

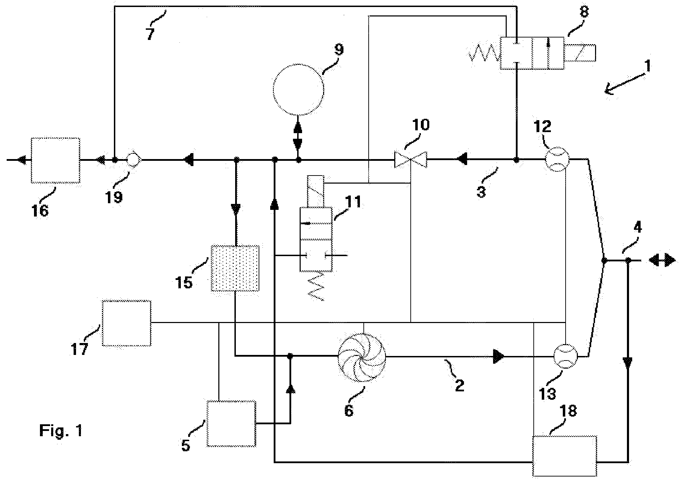

Figur 1 zeigt eine schematische Darstellung der erfindungsgemäßen Anästhesievorrichtung mit einem Atemkreis 1. Der Atemkreis 1 ist in Form eines Rückatemsystems ausgeführt, bei dem CO₂ von einem vom Patienten ausgeatmeten Atemgas durch einen CO₂-Absorber 15 entfernt wird. Im Anschluss wird dieses Atemgas wieder dem inspiratorischen Atemgas hinzugefügt. In einem derartigen Rückatemsystem wird nur das jeweils durch den Patienten verbrauchte Atemgas nachgeführt. Der restliche Anteil wird aus dem exspiratorischen Atemgas wieder verwendet. Die jeweiligen Atemgaspfade sind in der Figur 1 mit dicken Linien gekennzeichnet, wohingegen die dünnen Linien Steuer- und Datenleitungen zwischen den einzelnen Komponenten des Atemkreises 1 darstellen.

Von einer Atemgasversorgung 5 wird dem Atemkreis 1 Atemgas zugeführt. Das Atemgas wird in der Regel in der jeweiligen medizinischen Einrichtung aufbereitet und besteht im Wesentlichen aus einem Sauerstoff-Luft Gemisch, N₂O und volatilen Anästhesiegasen. Eine Atemgasfördereinrichtung 6 fördert dieses Atemgas über einen inspiratorischen Atemgaskanal 2 zu einem Y-Stück 4. In dem inspiratorischen Atemgaskanal 2 befindet sich ferner ein inspiratorisches Rückschlagventil (nicht dargestellt). An das Y-Stück 4 wird über einen Tubus (nicht dargestellt) der zu beatmende Patient angeschlossen. Das von dem Patienten ausgeatmete Atemgas wird während der Exspirationsphase wiederum über das Y-Stück 4 und einen exspiratorischen Atemgaskanal 3 über ein Peep Ventil 10 geführt. In dem exspiratorischen Atemgaskanal 3 ist ein exspiratorisches Rückschlagventil (nicht dargestellt) angeordnet. In dem Atemkreis 1 wird ferner das ausgeatmete Atemgas in einem Atemgasreservoir 9 zwischengespeichert. Dem Atemgasreservoir 9 ist ein CO₂-Absorber 15 nachgeschaltet. In dem CO₂-Absorber wird aus dem Atemgas CO₂ entfernt und anschließend das so gereinigte Atemgas wieder dem inspiratorischen Atemgaskanal 2 zugeführt.

Ist das Atemgasreservoir 9 hinreichend gefüllt, kann über ein Rückschlagventil 19 in ein an den Atemkreis 1 angeschlossenes Narkosefortleitungssystem 16 überflüssiges Atemgas abgegeben werden.

Sowohl in dem inspiratorischen Atemgaskanal 2 als auch in dem exspiratorischen Atemgaskanal 3 können mittels Drucksensoren die vorhandenen Atemgasdrücke überwacht werden (nicht dargestellt). Ein exspiratorischer Volumenstrom des Atemgases wird über einen ersten Volumenstromsensor 12 gemessen. Der Volumenstromsensor 12 ist im exspiratorischen Atemgaskanal 3 angeordnet. Ein zweiter Volumenstromsensor 13 befindet sich in dem inspiratorischen Atemgaskanal 2 und misst den Volumenstrom des inspiratorischen Atemgases. In einer Ausatmungsphase des Patienten (Exspirationsphase) stoppt die Atemgasfördereinrichtung 6 die Atemgasvolumenförderung und das Peep-Ventil 10 wird auf einen Endwert eines exspiratorischen Solldrucks eingestellt. Das im Patienten zwischengespeicherte Volumen kann dabei wieder in den Atemkreis 1 zurückfließen. Das so abgegebene Atemgasvolumen wird mittels des ersten Volumenstromsensors 12 gemessen und überwacht. Eine Konzentration des Atemgases wird bei einer Patientenbeatmung mittels einer

Atemgasmessvorrichtung 18 überwacht.

Der Atemkreis 1 der erfindungsgemäßen Anästhesievorrichtung enthält ferner einen Lufteinlass 11 zur Einleitung von Umgebungsluft in den Atemkreis 1. Während einer normalen Patientenbeatmung ist der Lufteinlass 11 geschlossen. Ebenfalls ist der Atemgasauslasskanal 7 durch ein geschlossenes Atemgasauslassventil 8 vom Atemkreis 1 getrennt. Im Fall eines zu hohen Atemgasdrucks kann das Atemgasauslassventil 8 geöffnet werden, um den zu hohen Atemgasdruck über den Atemgasauslasskanal 7 in das Narkosefortleitungssystem 16 abzuleiten. Mit der dargestellten erfindungsgemäßen Anästhesievorrichtung kann sowohl eine automatische Patientenbeatmung als auch eine manuelle Beatmung eines Patienten erfolgen.

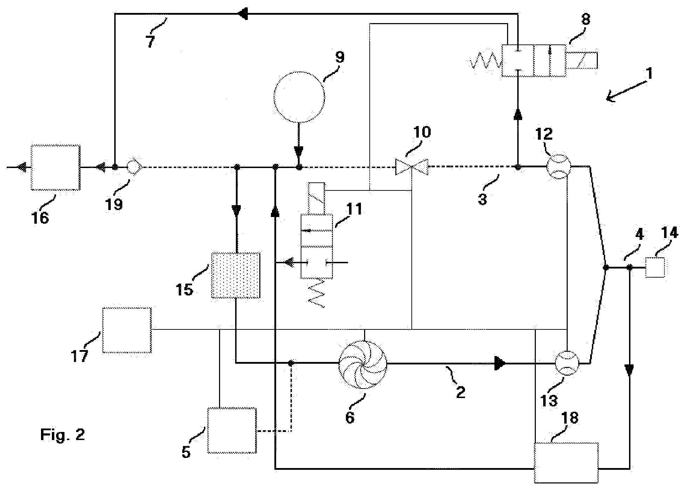

In Figur 2 ist das Prinzip des erfindungsgemäßen Verfahrens schematisch dargestellt. Dabei wird in einem ersten Verfahrensschritt das Y-Stück geschlossen. Hierzu wird befindet sich an dem Anästhesiegerät ein Dichtkonus 14. Auf den Dichtkonus 14 wird der patientenseitige Anschluss des Y-Stücks 4 gesetzt. Alternativ hierzu kann auch eine automatische Verschlussvorrichtung vorgesehen sein.

In einem nachfolgenden Verfahrensschritt wird das Peep-Ventil 10 geschlossen, so dass eine Verbindung zwischen dem Y-Stück 4 und dem Atemgasreservoir 9 über den exspiratorischen Atemgaskanal 3 unterbrochen wird. Die gesperrten Bereiche der Atemgaskanäle in dem Atemkreis 1 sind jeweils mit einer Strichlinie gekennzeichnet. Das Atemgasauslassventil 8 wird geöffnet, so dass eine Verbindung zwischen dem Y-Stück 4 und dem Atemgasauslasskanal 7 zu dem Narkosefortleitungssystem 16 entsteht. Im Anschluss wird der Lufteinlass 11 geöffnet und gleichzeitig oder in Folge die Atemgasfördereinrichtung 6 aktiviert. Damit wird Umgebungsluft von der Atemgasfördereinrichtung 6 angesaugt. Auf der Saugseite der Atemgasfördereinrichtung 6 wird dadurch ein Unterdruck erzeugt. Der Unterdruck führt dazu, dass zuerst das gesamte Atemgasvolumen aus dem Atemgasreservoir 9 herausbefördert und über den Atemgasauslasskanal 7 in das Narkosefortleitungssystem 16 befördert wird. Damit werden bereits die wesentlichen Bestandteile des Atemkreises 1 von Atemgas gereinigt.
Der Lufteinlass weist eine Nennweite von vorzugsweisen 5 bis 6 Millimeter auf. Mit diesen Dimensionen kann ein ausreichender Unterdruck für das Atemgasreservoir 9 erzeugt werden. Gleichzeitig kann man damit einen hinreichenden Volumenstrom gewährleisten. Das Narkosefortleitungssystem 16 weist eine maximale Aufnahme von mit Anästhesiemittel angereichertem Atemgas von 25 bis 50 Liter pro Minute auf. Die Atemgasfördereinrichtung 6 arbeitet mit diesen Kenngrößen mit einem Volumenförderstrom von 25 bis 50 Liter pro Minute.

Das erfindungsgemäße Verfahren bildet eine erste wesentliche Phase zur Reinigung von Atemgas des dargestellten Atemkreises 1, vorzugsweise zwischen der Behandlung von zwei Patienten.

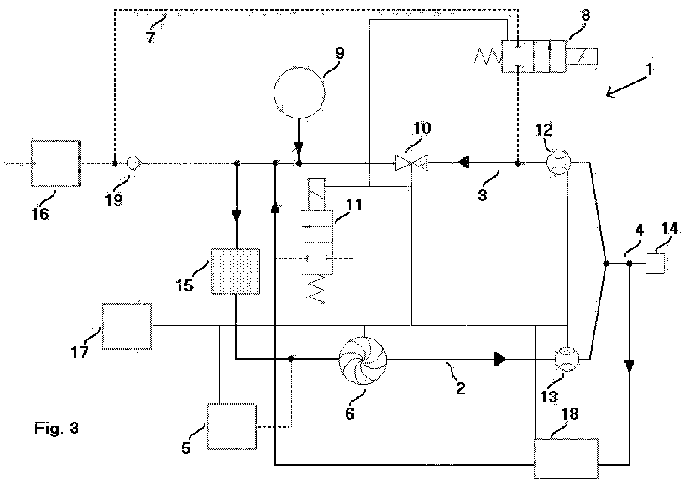

Um auch den Bereich zwischen dem Peep-Ventil 10 und dem Atemgasreservoir 9 des Atemkreises 1 von Atemgas zu reinigen, wird in einer weiteren Ausbildung des erfindungsgemäßen Verfahrens das Atemgasauslassventil 8 geschlossen und das Peep-Ventil 10 geöffnet. Dieser Zustand stellt eine zweite Phase der Aufbereitung des Atemkreises, dargestellt in Figur 3 dar. Als Umschaltpunkt zwischen der ersten und zweiten Phase kann ein vordefiniertes Atemgasvolumen oder eine Zeit verwendet werden. Eine Messung des Atemgasvolumens erfolgt vorzugsweise mit dem ersten Volumenstromsensor 12 im exspiratorischen Atemgaskanal. Der Beginn der Messung sowohl eines Atemgasvolumens als auch einer Zeit erfolgt nach dem Start der Atemgasfördereinrichtung 6. In vorteilhafter Weise beträgt ein vorbestimmter Wert des Atemgasvolumens 1 bis 2 Liter. Dieser Wert entspricht typischerweise dem zwei bis dreifachen Volumen an Atemgas des zu reinigenden Teils des Atemkreises 1. Die dazu benötigte Zeit bei einem Atemgasvolumen, angetrieben durch die Atemgasfördereinrichtung 6 von 50 bis 100 Liter pro Minute liegt bei ca. einer Sekunde. Im Anschluss daran wird vorzugsweise das erfindungsgemäße Verfahren in der Phase 1 nochmals durchgeführt werden.

Eine zwischen dem Atemgasreservoir 9 und dem Y-Stück 4 angeordnete Atemgasmessvorrichtung 18 kann die Konzentration des noch vorhandenen Atemgases bestimmen, so dass die Zyklen des erfindungsgemäßen Verfahrens in der Phase 1 und der weiteren Ausgestaltung des erfindungsgemäßen Verfahrens in der Phase 2 vorzugsweise so lange durchlaufen werden, bis eine gewünschte Konzentration an Atemgas erreicht wird.

Während der Aufbereitung des Atemkreises 1 saugt dabei die Atemgasmessvorrichtung 18 einen Teil des Volumenstromes ab, um somit auch diesen Atemgaspfad zu reinigen. Gleichzeitig kann die Atemgasmessvorrichtung 18 den Spülvorgang beziehungsweise dessen Wirksamkeit in Bezug auf eine Absenkung einer Atemgaskonzentration überwachen. Mit der Atemgasmessvorrichtung 18 kann somit die Anzahl der zu durchlaufenden Sequenzen des erfindungsgemäßen Verfahrens bestimmt werden. Alternativ hierzu kann der Anwender die Zeitdauer bzw. die Anzahl der Durchläufe des erfindungsgemäßen Verfahrens wählen.

Für einen Trocknungsvorgang des Atemkreises 1 wird ein Austausch der darin befindlichen feuchten Atemgase durch äußere trockenere Umgebungsluft erreicht. Für ein Austrocknen des Atemkreises 1 sind mehrere Zyklen des erfindungsgemäßen Verfahrens und gegebenenfalls der weiteren Ausgestaltung des erfindungsgemäßen Verfahrens in einer Phase 2 erforderlich. Ein Trocknungsvorgang des Atemkreises 1 ist in vorteilhafter Weise bei Beendigung des Einsatzes der Anästhesievorrichtung vorzunehmen. Dies kann zum Beispiel dadurch erfolgen, dass ein Therapeut oder Anästhesist den Trocknungsvorgang an der Anästhesievorrichtung startet. Die Anästhesievorrichtung kann dabei ausgebildet sein, nach einer vordefinierten oder einstellbaren Zeit auszuschalten.

Eine Beschleunigung des Trocknungsvorgangs kann durch eine Beheizung der Komponenten des Atemkreises 1 erfolgen. Dabei kann eine Heizungsvorrichtung eine höhere Heiztemperatur einstellen im Vergleich zu einem normalen Beatmungsmodus eines Patienten. Ein Vorteil des Trocknungsvorgangs mit Umgebungsluft im Vergleich zu den im Stand der Technik bekannten genutzten technischen Gasen liegt darin, dass eine gewisse Restfeuchtigkeit in der Umgebungsluft enthalten ist. Dies ist im Sinn einer schnellen Trocknung scheinbar von Nachteil, sichert jedoch, dass ein Kalk innerhalb des CO₂-Absorbers 15 nicht vollständig ausgetrocknet werden kann. Ein vollständig ausgetrockneter Kalk des CO₂-Absorbers 15 hat eine relativ geringe Standzeit im Sinne von CO₂-Absorption. Ferner hat er die Eigenschaft, volatile Anästhesiegase (insbesondere Isoflurane) aufzuspalten und dabei toxische Stoffe zu produzieren. Somit stellt die Nutzung von Umgebungsluft für den Austrocknungsvorgang des Atemkreises 1 der erfindungsgemäßen Anästhesievorrichtung mit Umgebungsluft eine wichtige Funktion in Hinsicht auf die Patientensicherheit dar.

In einer weiteren Ausgestaltung des erfindungsgemäßen Verfahrens kann durch eine Differenzbildung des ersten Volumenstromsensors 12 und des zweiten Volumenstromsensor 13 ein nicht korrekt geschlossenes Y-Stück erkannt und alarmiert werden. Dies erfolgt mittels inspiratorischer Volumenstrommessung, gemessen mit dem inspiratorischen zweiten Volumenstromsensor 13 abzüglich des mittels exspiratorischer Volumenstrommessung gemessenen exspiratorischen Volumenstroms. Wird diese Volumenstromdifferenz größer, beispielsweise 20 % des inspiratorischen Volumenstroms, so kann auf eine Leckage in dem Atemkreis 1 beziehungsweise auf ein nicht korrekt geschlossenes Y-Stück 4 geschlussfolgert werden. Dies kann dem Anwender in vorteilhafter Weise an der Anästhesievorrichtung angezeigt werden. Alternativ hierzu kann der Anwender die Zeitdauer bzw. die Anzahl der Durchläufe des erfindungsgemäßen Verfahrens wählen.

Eine weitere Optimierung des zeitlichen Bedarfs des erfindungsgemäßen Verfahrens kann an die jeweiligen spezifischen Volumina der jeweiligen Atemkreise 1 angepasst werden. Dabei kann das Volumen der entsprechenden Atemgaskanäle durch eine Compliance-Messung erfolgen. Die Compliance-Messung kann im Rahmen eines Einschalttestes durchgeführt werden. Somit kann die erfindungsgemäße Anästhesievorrichtung auf die jeweils vorhandenen variablen Patientenverbindungen angepasst werden. Eine benötigte Zeit für die Durchführung der Austrocknung des Atemkreises 1 kann auf empirisch ermittelten Daten oder auch auf einer in den Atemkreis 1 integrierte Feuchtigkeitsmessung basieren. Wird die Dauer der Trocknungsfunktion auf empirischer Basis ermittelt, so sollte die Zeitdauer erhöht werden mit der Dauer der Benutzungszeit des Atemkreises 1 seit der zuletzt durchgeführten Trocknungsfunktion. Je länger während dieser Benutzungszeit ein verhältnismäßig geringer Atemgasstrom durch die Atemgasversorgung 5 bereitgestellt wurde, umso höher sollte die Zeitdauer der Trocknungsfunktion gewählt werden.

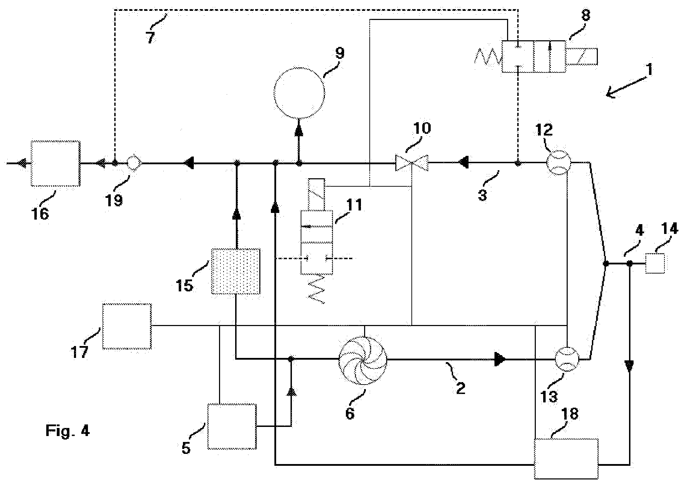

Für eine weitere Optimierung der Austrocknung des Atemkreises 1 kann trockenes Gas aus der zentralen Gasversorgung in den Atemkreises 1 eingespeist werden. Die Atemgasfördereinrichtung 6 ist dabei so eingestellt, das ca. die Hälfe des trockenen Gases durch den inspiratorischen Atemgaskanal 2 geführt wird. Die andere Hälfte wird entgegen der üblichen Atemgasrichtung durch den CO₂-Absorber 15 geführt. Sowohl der erste als auch zweite Gasanteil werden über das Rückschlagventil 19 in das Narkosefortleitungssystem 16 geführt (Fig. 4). Insbesondere nach Abschaltung der Heizung sinkt die Temperatur im Atemkreis 1, was eine erhöhte Kondensation verursacht. In vorteilhafter Weise wird diese Kondensation durch das eingeleitete trockene Gas vermieden.

Das erfindungsgemäße Verfahren kann im Rahmen einer Einschaltprozedur, zwischen der Anwendung der erfindungsgemäßen Anästhesievorrichtung zwischen zwei Patienten und während einer Ausschaltprozedur erfolgen.

### BEZUGSZIFFERN

- 1: Atemkreis
- 2: Inspiratorischer Atemgaskanal
- 3: Exspiratorischer Atemgaskanal
- 4: Y-Stück
- 5: Atemgasversorgung
- 6: Atemgasfördereinrichtung
- 7: Atemgasauslasskanal
- 8: Atemgasauslassventil
- 9: Atemgasreservoir
- 10: Peep-Ventil
- 11: Lufteinlass
- 12: Erster Volumenstromsensor
- 13: Zweiter Volumenstromsensor
- 14: Dichtkonus
- 15: CO₂-Absorber
- 16: Narkosefortleitungssystem
- 17: Steuervorrichtung
- 18: Atemgasmessvorrichtung
- 19: Rückschlagventil

## Patentansprüche

1. Anästhesievorrichtung mit einem Atemkreis (1) bestehend aus
einem inspiratorischem Atemgaskanal (2) und
einem expiratorischem Atemgaskanal (3) zur Aufnahme des ausgeatmeten Atemgases,
einem verschließbar ausgeführten Y-Stück (4) zur Verbindung des inspiratorischen und des expiratorischen Atemgaskanals (2, 3) mit einem Patienten,
wenigstens einer Atemgasversorung zur Einleitung von Atemgas in den Atemkreis,
einer Atemgasfördereinrichtung (6),
einem Atemgasauslasskanal (7) mit einem Atemgasauslassventil (8)
einem Atemgasreservoir (9) zur Zwischenspeicherung von Atemgas,
einer Steuereinrichtung (17),
**dadurch gekennzeichnet, dass**
ein Lufteinlass (11) zur Einleitung von Umgebungsluft in den Atemkreis (1) vorgesehen ist,
ein in Strömungsrichtung des Atemgases dem Atemgasreservoir (9) vorgeschaltetes Peep-Ventil (10) vorgesehen ist,
die Atemgasfördereinrichtung (6) in Strömungsrichtung des Atemgases dem Atemgasreservoir (9) nachgeschaltet ist,
der Lufteinlass (11) zwischen dem Peep-Ventil (10) und der Atemgasfördereinrichtung (6) angeordnet ist, und
die Steuereinrichtung zur Steuerung der durch den Lufteinlass (11) eintretenden Umgebungsluft, zur Steuerung des Peep-Ventils (10) und des Atemgasauslassventils (7) zur Reinigung des Atemkreises (1) von Atemgasen bei einem patientenseitig geschlossenen Y-Stücks (4) eingerichtet ist.

2. Anästhesievorrichtung nach Anspruch 1, **dadurch gekennzeichnet, dass** ein Dichtkonus (14) zum Verschließen des Y-Stücks (4) vorgesehen ist.

3. Anästhesievorrichtung nach einem der vorhergehenden Ansprüche, **dadurch gekennzeichnet, dass** der Lufteinlass (11) dem Atemgasreservoir (9) nachgeschaltet im Atemkreis (1) angeordnet ist.

4. Anästhesievorrichtung nach einem der vorhergehenden Ansprüche, **dadurch gekennzeichnet, dass** ein erster Volumenstromsensor (12) im Atemkreis (1) vorzugsweise im expiratorischen Atemgaskanal (3) vorgesehen ist.

5. Anästhesievorrichtung nach Anspruch 4, **dadurch gekennzeichnet, dass** ein zweiter Volumenstromsensor (13) im Atemkreis (1) vorzugsweise im inspiratorischen Atemgaskanal (2) vorgesehen ist, wobei ein geschlossenes Y-Stück (4) durch Differenzbildung der Werte der beiden Volumenstromsensoren (12, 13) ermittelbar ist.

6. Anästhesievorrichtung nach einem der vorhergehenden Ansprüche, **dadurch gekennzeichnet, dass** ein CO₂-Absorber (15) in dem Atemkreis (1) vorgesehen ist, der vorzugsweise dem Atemgasreservoir (9) nachgeschaltet ist.

7. Anästhesievorrichtung nach einem der vorhergehenden Ansprüche, **dadurch gekennzeichnet, dass** eine Atemgasmessvorrichtung (18) zur Überwachung der Konzentration an Anästhesiemittel in dem Atemgas vorgesehen ist, die vorzugsweise zwischen dem Y-Stück (4) und dem Lufteinlass (11) angeordnet ist.

8. Verfahren zum Betreiben einer Anästhesievorrichtung nach einem der vorhergehenden Ansprüche mit den folgenden Verfahrensschritten:
A: Schließen des Y-Stücks (4),
B: Schließen des PEEP-Ventils (10),
C: Öffnen des Atemgasauslassventils (8),
D: Öffnen des Lufteinlasses (11) und
E: Start der Atemgasfördereinrichtung (6),
so dass Umgebungsluft von der Atemgasfördereinrichtung (6) angesaugt wird und damit ein Atemgas aus dem Atemkreis (1) befördert wird.

9. Verfahren zum Betreiben einer Anästhesievorrichtung nach Anspruch 8, **dadurch gekennzeichnet, dass** ein Atemgasvolumen mit dem ersten Volumenstromsensor (12) und/oder eine Zeit im Anschluss an den Verfahrensschritt E gemessen werden.

10. Verfahren zum Betreiben einer Anästhesievorrichtung nach Anspruch 9, **dadurch gekennzeichnet, dass** nach dem Erreichen eines vorbestimmten Atemgasvolumens und/oder einer vorbestimmten Zeit das Atemgasauslassventil (8) geschlossen und das PEEP-Ventil (10) geöffnet wird.

11. Verfahren zum Betreiben einer Anästhesievorrichtung nach Anspruch 10, **dadurch gekennzeichnet, dass** ein Atemgasvolumen und/oder eine Zeit gemessen werden.

12. Verfahren zum Betreiben einer Anästhesievorrichtung nach Anspruch 11, **dadurch gekennzeichnet, dass** nach dem Erreichen eines vorbestimmten Atemgasvolumens und/oder einer Zeit wieder mit dem Verfahrensschritt A begonnen wird oder wenigstens das Y-Stück (4) geöffnet und der Lufteinlass (11) geschlossen wird.

13. Verfahren zum Betreiben einer Anästhesievorrichtung nach Anspruch 12, **dadurch gekennzeichnet, dass** der Volumenstrom wenigstens einem Volumen eines Teilstücks des expiratorischen Atemgaskanals (3) zwischen dem PEEP-Ventil (10) und dem Anschluss an dem Atemgasreservoir (9) entspricht.

14. Verfahren zum Betreiben einer Anästhesievorrichtung nach einem der Ansprüche 8 bis 13, **dadurch gekennzeichnet, dass** aus einer zentralen Gasversorgung trockenes Gas in dem Atemkreis (1) eingespeist wird, wobei vorzugsweise die Atemgasfördereinrichtung (6) so eingestellt wird, dass die Hälfte dieses Gases durch den inspiratorischen Atemgaskanal (2) geführt wird.

## Claims

1. Anesthesia device comprising a breathing circuit (1) consisting of
an inspiratory breathing gas duct (2) and
an expiratory breathing gas duct (3) for receiving expired breathing gas;
a closable Y-piece (4) for connecting the inspiratory breathing gas duct and the expiratory breathing gas duct (2, 3) with a patient;
at least one breathing gas supply for introducing breathing gas into the breathing circuit;
a breathing gas conveyor (6);
a breathing gas outlet duct (7) with a breathing gas outlet valve (8);
a breathing gas reservoir (9) for intermediately storing breathing gas;
a control device (17),
**characterized in that**
an air inlet (11) for introducing ambient air into the breathing circuit (1) is provided,
a PEEP valve (10) is provided in flow direction of the breathing gas upstream of the breathing gas reservoir (9),
the breathing gas conveyor (6) is arranged downstream of the breathing gas reservoir (9) in the direction of flow of the breathing gas,
the air inlet (11) is arranged between the PEEP valve (10) and the breathing gas conveyor (6), and
the control device is arranged for controlling the ambient air entering though the air inlet (11), for controlling the PEEP valve (10) and for controlling the breathing gas outlet valve (7) for removing breathing gases from the breathing circuit (1) if the Y-piece (4) is closed on the patient side.

2. Anesthesia device according to claim 1, **characterized in that** a sealing cone (14) is provided for closing the Y-piece (4).

3. Anesthesia device according to any of the preceding claims, **characterized in that** the air inlet (11) is arranged downstream of the breathing gas reservoir (9) in the breathing circuit (1).

4. Anesthesia device according to any of the preceding claims, **characterized in that** a first volume flow sensor (12) is provided in the breathing circuit (1), preferably in the expiratory breathing gas duct (3).

5. Anesthesia device according to claim 4, **characterized in that** a second volume flow sensor (13) is provided in the breathing circuit (1), preferably in the inspiratory breathing gas duct (2), wherein a closed Y-piece (4) can be detected by forming a difference of the values of the two volume flow sensors (12, 13).

6. Anesthesia device according to any of the preceding claims, **characterized in that** a CO₂ absorber (15) is provided in the breathing circuit (1), which CO₂ absorber is preferably arranged downstream of the breathing gas reservoir (9).

7. Anesthesia device according to any of the preceding claims, **characterized in that** a breathing gas measuring device (18) for monitoring a concentration of an anesthetic in the breathing gas is provided, which breathing gas measuring device is preferably arranged between the Y-piece (4) and the air inlet (11).

8. A method for operating an anesthesia device according to any of the preceding claims comprising the following steps:
A: closing the Y-piece (4),
B: closing the PEEP valve (10),
C: opening the breathing gas outlet valve (8),
D: opening the air inlet (11) and
E: starting the breathing gas conveyor (6),
such that ambient air is drawn in by the breathing gas conveyor (6) and breathing gas is delivered from the breathing circuit (1).

9. Method for operating an anesthesia device according to claim 8, **characterized in that** a breathing gas volume is measured with the first volume flow sensor (12) and/or a time is measured subsequent to method step E.

10. Method for operating an anesthesia device according to claim 9, **characterized in that** after reaching a predetermined breathing gas volume and/or a predetermined time, the breathing gas outlet valve (8) is closed and the PEEP valve (10) is opened.

11. Method for operating an anesthesia device according to claim 10, **characterized in that** a breathing gas volume and/or a time is measured.

12. Method for operating an anesthesia device according to claim 11, **characterized in that** after reaching a predetermined breathing gas volume and/or a time the method is started with method step A again or at least the Y-piece (4) is opened and the air inlet (11) is closed.

13. Method for operating an anesthesia device according to claim 12, **characterized in that** the volume flow corresponds at least to a volume of a portion of the expiratory breathing gas duct (3) between the PEEP valve (10) and the port to the breathing gas reservoir (9).

14. Method for operating an anesthesia device according to any of the claims 8 to 13, **characterized in that** dry gas is fed into the breathing circuit (1) from a central gas supply system, wherein the breathing gas conveyor (6) is set such that half of the dry gas is sent through the inspiratory breathing gas duct (2).

## Revendications

1. Dispositif d'anesthésie avec un circuit respiratoire (1) se composant de
un canal de gaz respiratoire inspiratoire (2) et
un canal de gaz respiratoire expiratoire (3) destiné à recueillir le gaz respiratoire expiré,
une pièce en forme de Y (4) pouvant être fermée, pour le raccordement du canal de gaz respiratoire inspiratoire et expiratoire (2, 3) à un patient,
au moins une alimentation en gaz respiratoire pour introduire du gaz respiratoire dans le circuit respiratoire,
un dispositif de propulsion de gaz respiratoire (6),
un canal d'échappement de gaz respiratoire (7) avec une soupape d'échappement de gaz respiratoire (8),
un réservoir de gaz respiratoire (9) pour le stockage intermédiaire de gaz respiratoire,
un dispositif de commande (17),
**caractérisé en ce qu'**il est prévu une entrée d'air (11) pour l'introduction d'air atmosphérique dans le circuit respiratoire (1),
il est prévu une soupape PEEP (10) disposée en amont du réservoir de gaz respiratoire (9) dans la direction d'écoulement du gaz respiratoire,
le dispositif de propulsion de gaz respiratoire (6) est disposé en aval du réservoir de gaz respiratoire (9) dans la direction d'écoulement du gaz respiratoire, l'entrée d'air (11) est disposée entre la soupape PEEP (10) et le dispositif de propulsion de gaz respiratoire (6), et
le dispositif de commande est conçu pour la commande de l'air atmosphérique entrant par l'entrée d'air (11), pour la commande de la soupape PEEP (10) et de la soupape d'échappement de gaz respiratoire (7) pour le nettoyage du circuit respiratoire (1) de gaz respiratoires lorsque la pièce en Y (4) est fermée côté patient.

2. Dispositif d'anesthésie selon la revendication 1, **caractérisé en ce qu'**il est prévu un cône étanche (14) pour la fermeture de la pièce en Y (4).

3. Dispositif d'anesthésie selon l'une quelconque des revendications précédentes, **caractérisé en ce que** l'entrée d'air (11) est disposée en aval du réservoir de gaz respiratoire (9) dans le circuit respiratoire (1).

4. Dispositif d'anesthésie selon l'une quelconque des revendications précédentes, **caractérisé en ce qu'**il est prévu un premier détecteur de courant volumique (12) dans le circuit respiratoire (1), de préférence dans le canal de gaz respiratoire expiratoire (3).

5. Dispositif d'anesthésie selon la revendication 4, **caractérisé en ce qu'**il est prévu un deuxième détecteur de courant volumique (13) dans le circuit respiratoire (1), de préférence dans le canal de gaz respiratoire inspiratoire (2), dans lequel une pièce en Y (4) fermée peut être déterminée en formant la différence entre les valeurs des deux détecteurs de courant volumique (12, 13).

6. Dispositif d'anesthésie selon l'une quelconque des revendications précédentes, **caractérisé en ce qu'**il est prévu un absorbeur de CO₂ (15) dans le circuit respiratoire (1), qui est de préférence disposé en aval du réservoir de gaz respiratoire (9).

7. Dispositif d'anesthésie selon l'une quelconque des revendications précédentes, **caractérisé en ce qu'**il est prévu un dispositif de mesure du gaz respiratoire (18) pour la surveillance de la concentration en agent anesthésiant dans le gaz respiratoire, qui est disposé de préférence entre la pièce en Y (4) et l'entrée d'air (11).

8. Procédé pour faire fonctionner un dispositif d'anesthésie selon l'une quelconque des revendications précédentes, comprenant les opérations suivantes:
A: fermeture de la pièce en Y (4);
B: fermeture de la soupape PEEP (10);
C: ouverture de la soupape d'échappement de gaz respiratoire (8);
D: ouverture de l'entrée d'air (11), et
E: démarrage du dispositif de propulsion de gaz respiratoire (6),
de telle manière que de l'air atmosphérique soit aspiré par le dispositif de propulsion de gaz respiratoire (6) et qu'un gaz respiratoire soit de ce fait transporté hors du circuit respiratoire (1).

9. Procédé pour faire fonctionner un dispositif d'anesthésie selon la revendication 8, **caractérisé en ce que** l'on mesure un volume de gaz respiratoire avec le premier détecteur de courant volumique (12) et/ou une durée à la suite de l'opération E.

10. Procédé pour faire fonctionner un dispositif d'anesthésie selon la revendication 9, **caractérisé en ce que**, après avoir atteint une volume de gaz respiratoire prédéterminé et/ou une durée prédéterminée, on ferme la soupape d'échappement de gaz respiratoire (8) et on ouvre la soupape PEEP (10).

11. Procédé pour faire fonctionner un dispositif d'anesthésie selon la revendication 10, **caractérisé en ce que** l'on mesure un volume de gaz respiratoire et/ou une durée.

12. Procédé pour faire fonctionner un dispositif d'anesthésie selon la revendication 11, **caractérisé en ce que**, après avoir atteint un volume de gaz respiratoire prédéterminé et/ou une durée prédéterminée, on recommence à l'étape A ou au moins on ferme la pièce en Y (4) et on ouvre l'entrée d'air (11).

13. Procédé pour faire fonctionner un dispositif d'anesthésie selon la revendication 12, **caractérisé en ce que** le courant volumique correspond au moins à un volume d'une portion du canal de gaz respiratoire expiratoire (3) entre la soupape PEEP (10) et le raccordement au réservoir de gaz respiratoire (9).

14. Procédé pour faire fonctionner un dispositif d'anesthésie selon l'une quelconque des revendications 8 à 13, **caractérisé en ce que** l'on introduit un gaz sec dans le circuit respiratoire (1) à partir d'une alimentation en gaz centrale, dans lequel on règle le dispositif de propulsion de gaz respiratoire (6) de telle manière que la moitié de ce gaz soit guidée à travers le canal de gaz respiratoire inspiratoire (2).
